# EUROPEAN PATENT APPLICATION

(11) **EP 4 464 790 A1**
(43) Date of publication of application: **20.11.2024**
(21) Application number: 23174056.4
(22) Date of filing: 17.05.2023
(51) Int. Cl.: C12Q 1/6806, C12Q 1/689

(54) **MOLECULAR TOOL FOR FAST DETECTION OF PATHOGEN**

(71) Applicant: daygnostics ag, 4057 Basel (CH)
(72) Inventor: Chevalier, Clément, 4123 Allschwil (CH); Braukmann, Fabian, 4051 Basel (CH); Whittle, Ingrid, 79576 Weil am Rhein (DE)
(74) Representative: P&TS SA (AG, Ltd.)

(57) **Abstract**

The present invention relates to an improved method and a kit for detecting and/or quantifying at least one of Listeria and Salmonella pathogens in a sample, comprising a selective enrichment step (S1), a sample preparation step (S2) including a step (S21) of suspending the cells in a liquid solution (L), a step (S22) of semi-specifically isolating the pathogens from the liquid solution (L) and a step (S24) of collecting the genetic material (G) of the pathogens (P1, P2); and a detection step (S3) including a real-time PCR (qPCR) detection of all the pathogens in a given sample.

## Description

### Technical domain

The present invention concerns a method for fast detection of a pathogen in a sample. More particularly, samples of food and food environment are concerned. The present invention also concerns a device for such a pathogen detection. The present invention further concerns a method and device for providing accurate data related to pathogen detection.

### Related art

Foodborne diseases caused by microbiological hazards are currently a growing public health concern (WHO, Geneva 2002). Most countries have documented significant increases in the incidence of diseases caused by microorganisms in food over the past few decades.

Listeria monocytogenes and Salmonella are pathogenic bacteria that can contaminate food products during or after processing (Cabedo, L et al. 2008). Salmonella cause illness every year in 1 out of 10 people and 33 million of healthy life years are lost. Most cases of salmonellosis are mild; however, sometimes it can be life-threatening. The severity of the disease depends on host factors and the serotype of Salmonella.

Listeriosis is one of the most serious severe foodborne diseases caused by the bacterium Listeria monocytogenes. Pregnant women, the elderly or individuals with a weakened immune system, such as people with immuno-compromised status due to HIV/AIDS, leukaemia, cancer, kidney transplant and steroid therapy, are at greatest risk of severe listeriosis and should avoid high risk foods. High risk foods include deli meat and ready-to-eat meat products (such as cooked, cured and/or fermented meats and sausages), soft cheeses and cold smoked fishery products. Listeria monocytogenes are widely distributed in nature. They can be found in soil, water, vegetation and the faeces of some animals and can contaminate foods.

Antimicrobial resistance is also a global public health concern and Salmonella is one of the microorganisms in which some resistant serotypes have emerged, affecting the food chain. Basic food hygiene practices, such as "cook thoroughly", are recommended as a preventive measure against salmonellosis.

Microbial testing is pillar in risk management of the food industry. It also allows to limit the use of antibiotics for treating patients afterwards. Food safety must be guaranteed in all foodstuffs. This creates a significant challenge for the pathogen tests, that need to be robust and sensitive in the wide range of different foodstuff, which differ in physical, chemical, and biological composition. Purifying and concentrating target pathogens remain an open challenge. The conventional methods for detecting the foodborne bacterial pathogens present in food are based on culturing the microorganisms on agar plates followed by standard biochemical identifications. Conventional methods can be time consuming as they depend on the ability of the microorganisms to grow in different culture media such as pre-enrichment media, selective enrichment media and selective plating media. Conventional methods require 2 to 3 days for preliminary identification and more than a week for confirmation of the species of the pathogens. Such a delay limits the application of the necessary tests for the food safety. Furthermore, it limits the possible preventive actions such as alerting or recalling the contaminated products early enough before its distribution.

Recently, different rapid methods with high sensitivity and specificity have been developed to deliver faster detection and identification of foodborne pathogens compared to conventional methods. One of the most used molecular-based method for the detection of foodborne bacterial pathogens is quantitative polymerase chain reaction (qPCR). This sensitive method has been used to detect Salmonella and Listeria monocytogenes and Listeria spp. Despite theses recent developments, properly isolating the pathogens out of a food sample remain a challenging task. Also the obtained results still need to be properly decrypted, which is not always an easy task. The overall analytical time thus remains perfectible.

There is still room to improve the detection and the quantification of food pathogens, in particular Listeria and Salmonella, in a fast and reliable routine operation. There is more specifically a need to overall better and faster identify the pathogens present in a food sample so that a rapid decision of conformity or non-conformity can be taken.

### Short disclosure of the invention

An aim of the present invention is the provision of a method that overcomes the shortcomings and limitations of the state of the art. It is in particular an aim to provide a method that improves at least one of the sensitivity, the rapidity, the reproducibility, the reliability of the pathogen quantification and/or detection of a sample. It is another aim of the present invention to quantify and/or detect more than one pathogen in a sample. In particular, such a quantification and/or detection of several pathogens are possible through a single operation. It is a further aim of the present invention to facilitate the reading of the obtained results for a faster and/or a more reliable decision concerning the conformity of a sample, in particular a food sample. The present method aims more specifically at better distinguishing between false positive or negative and true results, and or between several pathogens. It is a further aim of the present invention to facilitate a systematic and routine test process for samples, in particular food samples.

Another aim of the invention is to provide a kit adapted for routine and efficient test at points of production or another logistic points of foodstuff.

According to the invention, these aims are attained by the object of the independent claims, and better defined in the dependent claims.

With respect to what is known in the art, the invention provides the advantage that an easier and a faster routine test is possible.

### Short description of the drawings

Exemplar embodiments of the invention are disclosed in the description and illustrated by the drawings in which:

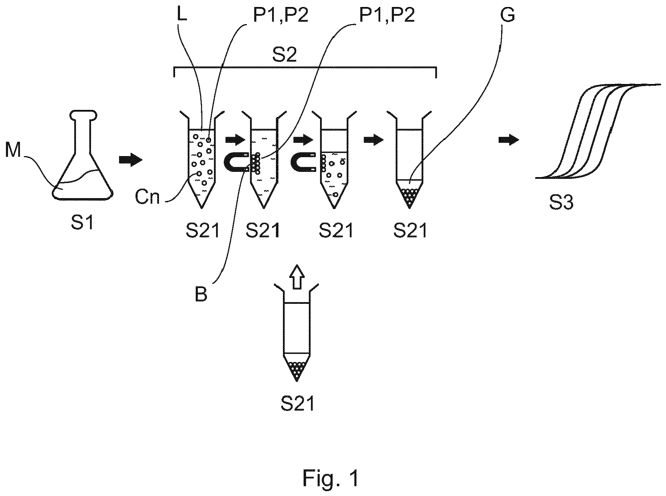

- Figure 1 : Schematic view of the detection process according to an example of the present disclosure.
- Figure 2a : experimental results of Salmonella culture in different culture media.
- Figure 2b: experimental results of Listeria monocytogene culture in different culture media.
- Figure 3a, 3b: experimental results of Listeria ivanovii on different culture media.
- Figure 4a: experimental results on detection of Salmonella with various microbeads.
- Figure 4b: : experimental results on detection of Listeria with various microbeads.
- Figure 4c, 4d: comparative results related to several microbeads.
- Figure 5: experimental results on buffering agents.
- Figure 6: experimental results on digesting agents.
- Figures 7a, 7b: experimental results on 2 step detection process.
- Figure 8: experimental results on dyes for sample detection.
- Figures 9a, 9b: experimental results on dilution experiments.
- Figure 10: experimental results on internal control.
- Figures 11: experimental results on the effect of BSA.
- Figures 12a, 12b, 12c: experimental results on multiplex detection.

### Examples of embodiments of the present invention

The described method is adapted to detect the presence of at least one pathogen **P1, P2** in a sample. A sample denotes here a food sample, including any natural food such as vegetables, fruits, meat, fish, eggs and related products. A sample also denotes any part of a transformed food stuff such as an intermediate or semi-finished food product, deli, cheese, precooked meal, row material included in food stuff preparation, and any related product. A sample also denotes any liquid production including milk, soda, beverages, cream, sirup, precooked sauces or preparations, and any related products. A sample denotes in addition any material taken from the environment of a food preparation installation or premise, being individual, collective or industrial, in particular for sanitary inspection or control. Such material can be sampled out of surfaces or can consider tools or cooking material such as oven and related objects. Sample can thus be taken from a kitchen of an individual, from a food truck, a restaurant or a take away booth, a cafeteria or a collective food provider for hospitals or schools. It can also be taken along any logistic point of a distribution including a food industry, a food distributor, a supermarket, a food storage unit, a food conditioning unit, a food transport vehicle and so on.

The method can easily be applied for a collection and preparation of a sample at a point of care.

It is furthermore adapted to target some well known pathogens so that their detection can be fast, non expensive and reliable. This allows for example a systematic or semi-systematic control at certain key points. For example, when delivering or receiving a food stuff, in particular at industrial premises. The method typically serves as quality control although it can be applied for research investigations. Among the known pathogens, the method is preferably applied to most dangerous and/or frequent pathogens including pathogens responsible for Listeria and salmonella. The method preferably targets the corresponding bacteria so as to identify the related genetic material.

A pathogen **P1, P2** is thus preferably selected among bacteria responsible for Listeria and/or Salmonella. Pathogens include for example *Listeria ivanovii, Listeria monocytogens, Salmonella typhi, Salmonella typhimurium, Salmonella paratyphi, Salmonella enteritidis.*

The present method is preferably adapted to detect several of the targeted different pathogens **P1, P2** present in a given sample. In other word, a single detection allows to identify different pathogens so that the sample preparation time remains as short as possible, while remaining accurate.

The present method allows to detect the targeted known pathogen with an improved accuracy and/or rapidity. To this end, it comprises at least one step, preferably a combination of steps, allowing to significantly improve the selectivity of the pathogen detection, with regard to the potential other material present in the sample, including numerus non pathogen cells.

The method thus comprises an enrichment step **S1** wherein the targeted known pathogens **P1, P2** are allowed to grow. According to an embodiment, the targeted known pathogens **P1, P2** grow in a proper way to be detected. According to another embodiment, the targeted known pathogens **P1, P2** grow faster than the other cells of the sample, which are not object of the detection. To this end, the sample is placed on a selective culture medium **M,** adapted to the targeted known pathogen. Such a culture medium can be selected among a culture medium based on a mixture of ½ Fraser and tryptic soy Broth (TSB). Alternatively, the culture medium **M** can be based on a Brain Heart infusion Broth (BHI) or on a mixture of ½ Fraser and BHI. Alternatively, the culture medium **M** can be a buffered pepton water (BPW). These culture media have been found to be particularly suitable for the bacteria responsible for the diseases listeriosis and / or salmonellosis.

It is noted that the culture medium **M** is selected so as to be suitable for all the targeted known pathogens. The culture medium **M** is defined so that both Listeria and Salmonella are selectively grown.

The term "selective", "selectively" and related terms designate a positive discrimination between the considered material and at least one of the surrounding material. While the considered material is privileged compared to at least one of the surrounding material, is can still be contaminated with some part of the surrounding material. The selectivity applies to one single target material or to a combination of several targeted materials, such as the Listeria and Salmonella pathogens. After a selective operation, such as a selective culture or a selective isolation or a selective detection, the targeted pathogen or the targeted pathogens are over expressed compared to at least some surrounding material.

The terms "specific" or "specifically" and related terms designate a complete positive discrimination between a targeted material and the surrounding material. In other word, a specific operation such as a specific culture, a specific isolation or a specific detection allows to express only the targeted pathogen. The term "specific", "specifically" and related terms thus applies by nature to a unique type of material. It preferably applies to one unique pathogen.

The term "semi-specific" and related terms denotes a complete positive discrimination of more than one targeted material. For example, a semi-specific operation such as a semi-specific culture, a semi-specific isolation or a semi-specific detection allows to express only the targeted pathogens of a given sample or various pathogens of a given type of pathogens.

The culture medium **M** of step **S1** is selected to specifically, semi-specifically or selectively increase the amount of a targeted known pathogen or a selection of targeted known pathogens **P1, P2** with regards to the other cells of the sample, or at least part of them. More preferably, the culture media M is defined so as to semi-specifically or selectively increase the amount of a targeted known pathogen or a selection of targeted known pathogens **P1, P2** with regards to the other cells of the sample, or at least part of them. The culture medium **M** thus comprises nutritive elements which allow increasing the growth of the targeted pathogens compared to the other cells of the samples.

According to an embodiment, the culture medium **M** further comprises elements which selectively, semi-specifically or specifically degrade some cells also present in the sample, or at least prevents their growth. Such preventing element can be selected among any known active material such as chemical compounds like salts including metal salts, small organic molecules, pH adjusting compounds like NaOH, KOH, HCl, Na₂HPO₄, NaH₂PO₄, biological material like antigens, peptides, proteins. The preventing elements can be selected among an antibiotic or a combination of antibiotics adapted to kill or prevent growth of at least some cells others than the targeted pathogens. Examples of preventing elements are Lithium chloride LiCl, Nalidixic acid, Acriflavine.

Examples of selective, semi-specific or specific culture media M are BHI, BHI90% + ½ F 10%, BHI 66% + 1/2F 35%, BHI SSS 1:3, BHI SSS 1:10, TSB, TSB 90%+1/2F 10%, TSB SSS 1:3, TSB SSS 1:10. These exemplified media M can be free of any antibiotic or comprise on the above mentioned preventing compounds, in particular a suitable amount of LiCI, Nadilixic acid, Acriflavine or a combination thereof.

The culture conditions are adapted to properly grow the cells. The temperature is determined in a proper range. Suitable temperature is for example comprised between 25°C to 40°C, preferably around 30°C to 38°C.

The time of culture is also determined to increase the number of targeted known pathogen. It is preferably determined to also increase the ratio of the known targeted pathogens compared to at least some of the other cells of the sample. It is typically around few hours such as 5 to 20 hours, or 8 to 15 hours. The composition of the culture medium **M** and the culture conditions are preferably defined to fast growing the targeted pathogens so that the culture time remains as short as possible, preferably below 24h or below 15 hours or below 10 hours.

According to an embodiment, additional agents such as buffering agents can be added to the culture medium **M.** Buffering agent such as AGT-1 or TTGB or a combination thereof.

The enrichment step **S1** thus provides a mixture of cells comprising the targeted known pathogen or pathogens **P1, P2** together with some other cells Cn which are not assumed to be analysed. The other cells Cn are thus considered as contaminating material.

The present method comprises a sample preparation step **S2.** The sample preparation step **S2** aims at properly conditioning the targeted known pathogen or the targeted known pathogens **P1, P2** so that they can be detected. The sample preparation step **S2** also allows to further improve the ratio of the known targeted pathogen or pathogens **P1, P2** in the analytical sample resulting from the sample preparation, compared to the other cells Cn or at least part of them. In other words, the sample preparation allows to selectively or semi-specifically preferably semi-specifically purify and concentrate said targeted known pathogen or pathogens **P1, P2.**

To this end, the sample preparation step **S2** a step **S21** of suspending the cells of the culture medium **M** after the enrichment step **S1** in a suitable liquid solution L. The liquid solution L is adapted to collect the cells of the culture medium **M** and maintain them in suspension while preserving their viability. It is preferably an aqueous solution, which comprise some salts and/or other suitable components.

The sample preparation step **S2** further comprises an isolation step S22 allowing to isolate the targeted known pathogen or pathogens **P1, P2** from the liquid solution L. The targeted pathogen or pathogens are preferably semi-selectively isolated out of the liquid solution L. To this end, the content of the liquid solution L, including the targeted pathogen or pathogens **P1, P2** is contacted with a solid support the surface of which comprises a semi-selective binder, adapted to semi-selectively bind the targeted pathogen or pathogens. The solid support can be of any suitable kind. It can be for example some filtration device allowing to filter out the liquid L and retain the targeted pathogen or pathogens. Alternatively, it can be solid material which can be immersed in the liquid solution L so as to allow the targeted pathogen or pathogens to bind. Such a solid support can take the form of a porous media having a suitable surface of contact, or pellets, or membranes, or particles, or beads, or microbeads.

According to an embodiment, the liquid solution L is filtered so as to retrieve the solid support combined with the targeted pathogen or pathogens **P1, P2.** Alternatively or in addition, a centrifugation step is applied so as to better separate the solid support from the rest of the material.

According to another embodiment, the solid support comprises a mean adapted to retrieve it out of the liquid solution L. In particular, in case no filtration is applied during the sample preparation, then, the targeted pathogen or pathogens can still be easily retrieved out of the liquid solution L. This is also applicable when a centrifugation step is applied, wherein the solid support remains in presence of some contaminating material. As an example, the solid support comprises a magnetic sensitive material so that it can be isolated out of the liquid solution under a magnetic field.

According to a preferred embodiment, the solid support is or comprises magnetic beads or magnetic microbeads **B,** coated with at least one binder semi-specific from the targeted pathogen or pathogens. Such a magnetic beads or microbeads **B,** can be collected out of the liquid solution L when applying a suitable magnetic field or force.

The targeted pathogen or pathogens **P1, P2** are covalently bond to the solid support. The binder or the combination of binders arranged at the surface of the solid support can be of any kind of semi-specific material. In other words, the binder or the combination of binders of the solid support allows to bind all the targeted pathogen or pathogens while remaining none reactive, or essentially non-reactive, towards the other cells Cn contaminating them.

The binder can be selected among any suitable protein, protein fragment, antibody, peptide, either natural or chemically modified, as long as it is adapted to semi-specifically bind the targeted pathogen or pathogens **P1, P2.** The binder can alternatively be of the type of DNA, RNA or fragments thereof or related material.

The binder is preferably selected so as to allow a multiplexing of several targeted pathogens. In particular, the binder is selected so as to allow the binding of at least one salmonella and at least one Listeria's related pathogens.

Examples of solid supports are microbeads B such as rapiprep^{®}, designating ApoH technologies Peps6 magnetic beads, dynabeads^{®}, Designed as DNA direct magnetic beads, Anti-Salmonella magnetic beads, or anti-Listeria magnetic beads.

The solid support is preferably a microbead or mixture of microbeads coated with an Apoh, an Apoh derivative or fragment, or any related complex and/or mixture. ApoH here designates Apolipoprotein H. According to a preferred embodiment, the binder is Peps6.

The binding of the targeted pathogen or pathogens in the step **S22** is preferably followed by a releasing step **S23,** wherein the targeted known pathogen or pathogens **P1, P2** are separated from their solid support, in an appropriate liquid composition, for further treatment.

The sample preparation step **S2** further comprises a collecting step S24 aiming at collecting the genetic material G or part of it related to the targeted known pathogen or pathogens **P1, P2.** The collection step **S24** can be performed by any suitable means allowing to extract the genetic material G from the targeted pathogen or pathogens **P1, P2.** Such means can be a chemical, a mechanical, a thermal treatment or a combination thereof. For example, a chemical treatment including a pH adjustment using NaOH, KOH, HCl, H₃PO₄ or related pH adjustment agent can be applied. Alternatively or in addition, a centrifugation can also be applied so as to free the genetic material of the targeted pathogen or pathogens. Alternatively or in addition, a thermal treatment such as an increase of the temperature up to 40°C, 45°C, 50°C or higher, such as up to 110°C or 100°C for a suitable time can be applied. For example, temperatures comprised between around 40°C and around 100°C or 98°C, or between 50°C and 90°C can be applied.

It is understood that the collecting step **S24** comprises the lysis of the targeted pathogen or pathogens **P1, P2,** allowing to retrieve the corresponding genetic material G. The genetic material denotes any DNA or RNA content of the targeted pathogen or pathogens under any form.

The sample preparation step **S2** can optionally comprise additional steps such as intermediate thermal, mechanical or chemical treatments depending to the needs. Fore example, an intermediate centrifugation step or sedimentation step **S25** can be included in the sample preparation process. Alternatively or in addition, at least one of the enrichment step **S1,** sample preparation step **S2** and detection step **S3,** better described below can include buffering conditions resulting from the presence of one or more buffer. A buffer can be selected among AGT-1, TTGB and any other suitable buffering agent or composition.

Alternatively or in addition, at least one of the enrichment step **S1** and sample preparation step **S2** comprises a digesting step of degrading any organic matrix such as food matrix, vegetal fibres, and any related organic structure. Such a digestive step allows to better disperse the targeted known pathogen or pathogens in the culture medium, or in the analytical sample. Depending on the nature of the sample, various digestive components or composition can be used. The digestion can be based on chemical treatment, such as acids or basic treatments, optionally combined with temperature increase. The digesting step can alternatively be based on a biodegradation with active biopolymers such as enzymes or enzyme compositions. As an example, a digesting step can be operated in the presence of a protease such as an alkaline protease (APA) or Trypsin or a combination thereof.

The present method further comprises a detection step **S3** adapted to detect and identify the genetic material G of the targeted pathogen or pathogens. The detection step includes a polymerase chain reaction (PCR) detection, in particular a real-time PCR detection. The genetic material G collected from the collecting step **S24** is purified and amplified based on a suitable primer set.

The detection step **S3** allows to provide data related to the presence and/or amount of at least one of the targeted known pathogens **P1, P2.** The detection preferably allows to simultaneously detect the presence of several targeted known pathogen or pathogens, thanks to the multiplexing properties of the solid support, in particular the magnetic bead **B,** used in the isolation step **S22.**

According to an embodiment, the detection step **S3** comprises a short 2 step temperature program comprising a first temperature level T1 and a second temperature level **T2** lower than the first temperature level T1. In the first temperature level **T1,** the temperature is maintained between 90°C and 100°C for a suitable time. In the second temperature level **T2,** the temperature is maintained between around 55°C and around 75°C or 65°C for a suitable period of time. The 2 step temperature program is well adapted for a fast detection. The global detection time, comprising the first **T1** and the second temperature levels **T2,** is preferably lower than 1 hour, preferably lower than 45 minutes, more preferably lower than 30 minutes. It is for example comprised between 10 to 30 minutes.

The detection step **S3** is adapted to well differentiate the targeted pathogens present in the analytical sample. It is further well adapted to detect the targeted pathogens with an improved accuracy so as to avoid or at least limit the risk of mistakes. Most of the used qPCR reagents are transparent, which makes them difficult to see, in particular at a point of care. Furthermore, known dyes currently used for the qPCR, such as Biorad - Precision Blue Real-Time PCR Dye, negatively impact the performances of the qPCR. In the present method, , a step of incorporating one or several dyes in the analytical sample, is included. The dyes are selected so as to not negatively impact the performances of the qPCR, while increasing the visibility of the detection reagents. The dyes are furthermore preferably each selective of a given targeted pathogen. This results in a coloration of the analytical solution, which would otherwise remain transparent. The resulting colour is detected by means of any appropriate optical detector. In particular, wavelengths and intensities of the corresponding light can be detected, collected and stored into appropriate digital means. The detection step S3 allows to independently detect several pathogens without negatively impacting the real time PCR process. A dye can be of any suitable kind, such as fluorescent, phosphorescent or not, being active in the visible range, in the infrared range or in the UV range.

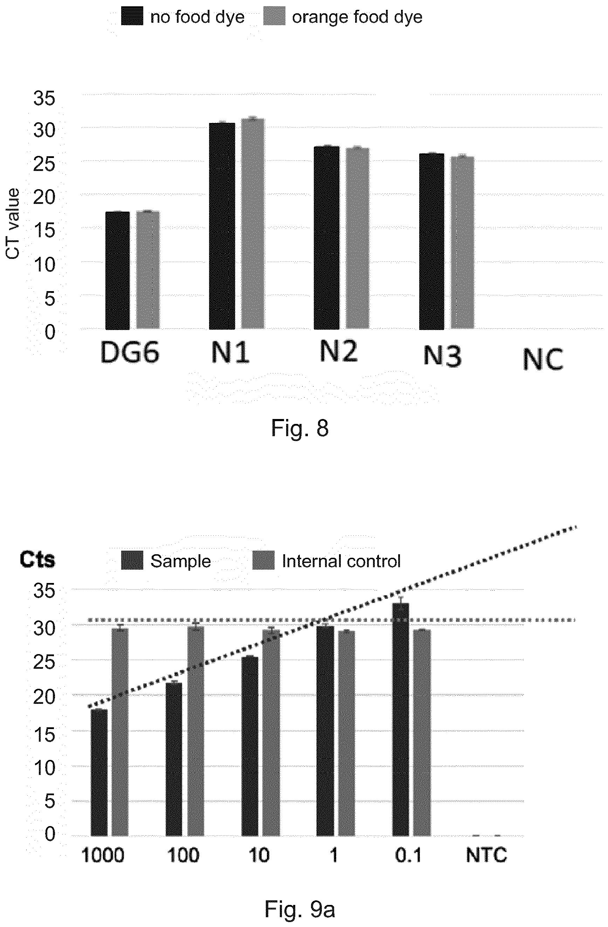

It has been found that the following dyes or mixtures of dyes allowed to increase user-friendliness and robustness without compromising the qPCR performance. In particular, a mixture of E104 (Quinoline Yellow WS), E110 (orange yellow S), E124 (Ponceau 4R), (E122 (Azorubine), E129 (Allura Red AC), E133 (Brilliant blue FCF) allowed to reach such an improved result. Also, mixtures of yellow or orange appearance containing different ratios of E104 (Quinoline Yellow WS), E110 (orange yellow S), E124 (Ponceau 4R), did not compromise the qPCR performance and increased visibility. Figure 8 shows the corresponding comparative results, with and without these dyes.

According to an embodiment, the detection step **S3** comprises detection of several samples having different dilution levels of purified DNA so as to detect low to high amount of said targeted pathogen or pathogens.

The detection step **S3** may further comprise the incorporation and the detection of an internal control probe used as a positive control. Any suitable probe can be used to confirm that the detection occurs under optimal conditions.

The present method further comprises a step **S4** of computing the data resulting from the detection step **S3** so as to provide a reliable an reproducible result. In particular, the data is computed so as to automatically correct potential defects. For example, specific optical thresholds of detection can be set so as to better determine the presence or absence of a targeted pathogen in the analytical sample. Other parameters such as wavelengths selection or filtering can also be considered. This minimizes or avoids the variability of interpretation of an operator. The data is thus automatically computed based on predetermined parameters and/or algorithms so as to provide a final result to the operator. The final result can be provided by means of any suitable device, such as a display or a computer screen, a printed paper, a remote device, for example a patient's device, like a mobile phone or a terminal.

According to an embodiment, the operator can adjust one or several parameters so that the final results better discriminates between presence or absence of a pathogen or pathogens.

According to another embodiment, the final results can be stored in a memory for further analysis or computation, or for feeding databases. According to another embodiment, the final results can be further treated through an artificial intelligence or a deep learning module.

The present invention further covers a kit adapted to detect and/or quantify at least one targeted known pathogen in a sample. In particular, the targeted known pathogen or pathogens are selected among the above mentioned pathogens. Advantageously, the present kit allows an easy test at a point of care, such as a production line or a logistic point.

According to an embodiment, the kit comprises one or several enrichment media **M,** as above defined. The kit is in particular adapted for detecting a targeted known pathogen or several pathogens. The culture media **M** are then adapted accordingly. The culture media **M** can be packed in individual and sterile bags or containers. The culture media **M** can be accompanied by indications related to the type of pathogen they are best suitable for. Any additional relevant information such as time and temperature of culture can also be provided.

The kit according to the present disclosure further comprises at least one discriminant agent adapted semi-specifically isolate the targeted known pathogen or pathogens **P1, P2.** Such a discriminant agent can be a solid support above defined. In particular, the discriminant agent is specifically adapted for the pathogen or pathogens for which the kit is dedicated. The above magnetic beads **B** can thus be provided. The discriminant agent can be separately packed in a sealed bag or container.

### Examples

### Salmonella culture

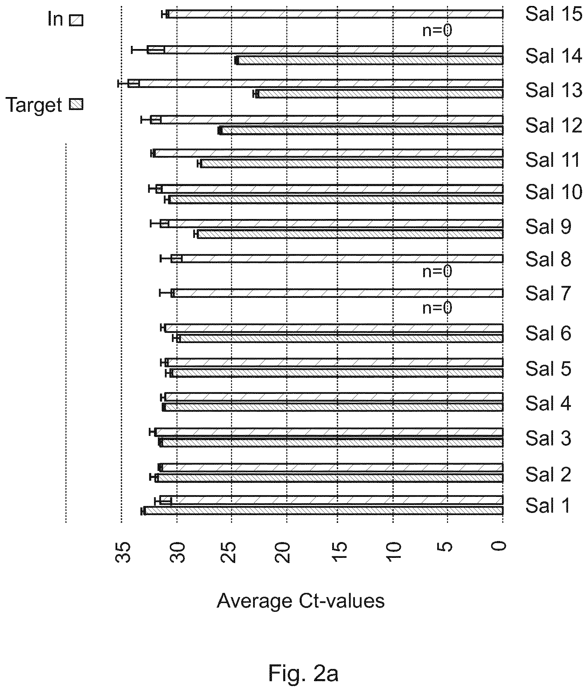

Salmonella was grown in the following different culture media and detected by qPCR with an internal control. The corresponding results are given in figure 2a.

| **Sample** | **Culture media** | **Time of culture** |
|---|---|---|
| Sal1 | SA + Meat BPW 1 | 8h |
| Sal2 | SA + meat BPW 2 | 8h |
| Sal 3 | SA + meat BHI 1 | 8h |
| Sal4 | SA + meat BHI 2 | 8h |
| Sal5 | SA + meat TSB 1 | 8h |
| Sal 6 | SA + meat TSB 2 | 8h |
| Sal 7 | C- meat BPW | 10h |
| Sal 8 | C-meat BHI | 10h |
| Sal 9 | SA + Meat BPW 1 | 10h |
| Sal 10 | SA + Meat BPW 2 | 10h |
| Sal 11 | SA + meat BHI 1 | 10h |
| Sal 12 | SA + meat BHI 2 | 10h |
| Sal 13 | SA + meat TSB 1 | 10h |
| Sal 14 | SA + meat TSB 2 | 10h |
| Sal 15 | C- meat TSB | 10h |

Wherein
meat denotes minced meat beef
TSB1/TSB2 denote different replicates of Tryptic soy broth having the following composition:
   Tryptone :17 g
   Soytone : 3 g
   Dextrose : 2.5 g
   NaCl : 5.0 g
   K₂HPO₄ : 2.5 g
BPW1/ BPW2 denote different replicates of Buffered peptone water having the following composition:
   Peptone : 10 g
   Sodium chloride : 5 g
   Disodium phosphate : 3.5 g
   Mono-potassium Phosphate :1.5 g
   Distilled water: 1 L
BHI1/BHI2 denotes different replicates of Brain heart infusion having the following composition:
   beef heart (infusion from 250g) : 5 g/L
   calf brains (infusion from 200g) : 12.5 g/L
   disodium hydrogen phosphate : 2.5 g/L
   D(+)-glucose : 2 g/L
   peptone : 10 g/L
   sodium chloride : 5 g/L

### Listeria culture

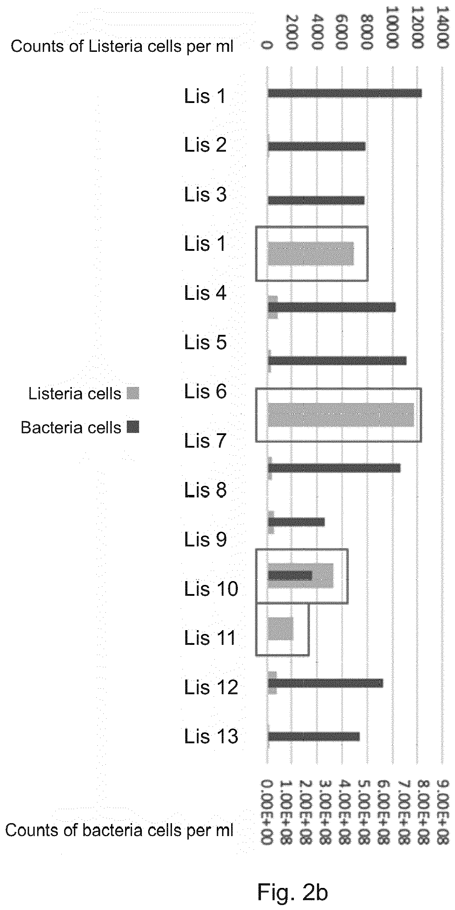

Listeria monocytogenes was grown in the following media in the presence of other bacteria. The culture media were then screened. The corresponding results are provided in figure 2b.

**Table 1**

| sample | Culture media |
|---|---|
| Lis 1 | BHI |
| Lis 2 | BHI 90% - 1/2F 10% |
| Lis 3 | BHI 66% - 1/2F33% |
| Lis 4 | BHI SSS |
| Lis 5 | BHI SSS 1: 3 |
| Lis 6 | BHI SSS 1:10 |
| Lis 7 | ½ F |
| Lis 8 | TSB |
| Lis 9 | TSB 90% - 1/2F 10% |
| Lis 10 | TSB 66% - 1/2F 33% |
| Lis 11 | TSB SSS |
| Lis 12 | TSB SSS 1 : 3 |
| Lis 13 | TSB SSS 1 : 10 |

Wherein
TSB and BHI are as above-defined and
SSS denotes a selective supplement having the following composition :
   LiCI : 3 g/l
   Nalidixic acid : 0.01 g/l
   Acriflavine : 0.0125 g/l
1/2F denotes the following composition :
   Proteose peptone (peptic digest of animal tissue) : 5.0g
   Tryptone (pancreatic digest of casein) : 5.0g
   Beef extract: 5.0g
   Yeast extract: 5.0g
   Sodium chloride: 20.0g
   Di-sodium hydrogen orthophosphate .2Hz0 : 12.0g
   Potassium di-hydrogen orthophosphate : 1.35 g
   Aesculin : 1g
   Lithium chloride : 3.0g
   Iron (III) ammonium citrate : 0.5g
   Nalidixic acid : 0.01g
   Acriflavine : 0.0125g
   Distilled or deionized water :1000.0 ml

### Enrichment with antibiotics:

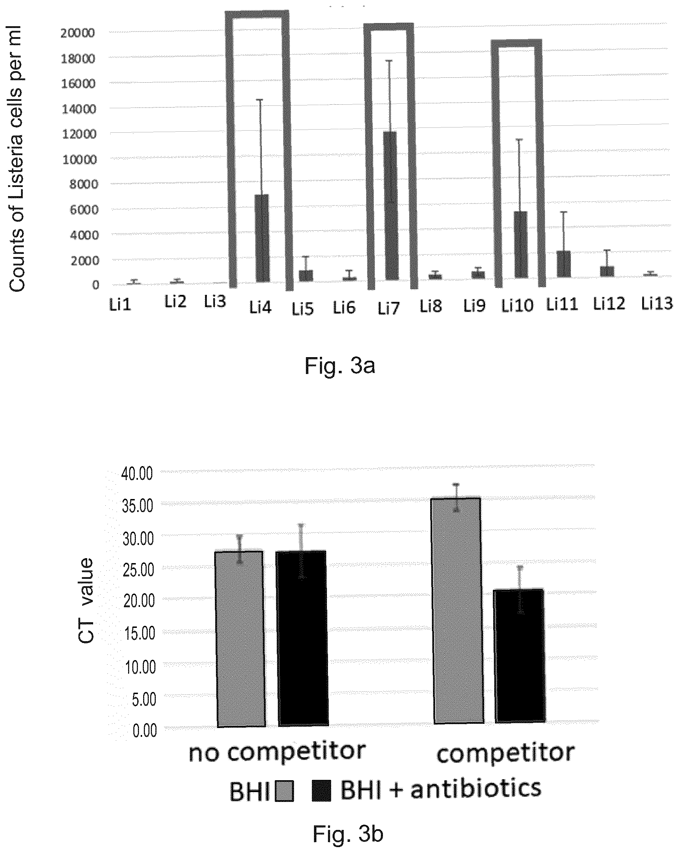

Listeria ivanovii was grown in the following culture media in the presence of competing bacteria. The cell counts was performed 13.5 hours after growth on RAPID'L.mono Agar Plates. The corresponding results are provided on figure 3a.

**Table 2**

| sample | Culture media |
|---|---|
| Li 1 | BHI |
| Li 2 | BHI 90% - 1/2F 10% |
| Li 3 | BHI 66% - 1/2F33% |
| Li 4 | BHI SSS |
| Li 5 | BHI SSS 1: 3 |
| Li 6 | BHI SSS 1:10 |
| Li 7 | ½ F |
| Li 8 | TSB |
| Li 9 | TSB 90% - 1/2F 10% |
| Li 10 | TSB 66% - 1/2F 33% |
| Li 11 | TSB SSS |
| Li 12 | TSB SSS 1 : 3 |
| Li 13 | TSB SSS 1 : 10 |

Listeria ivanovii was grown 13.5 hours in swabs on BHI and BHI + antibiotics, with or without lactobacillus caseii as a competitive bacteria. The antibiotics are lithium chloride, Nalidixic acid and Acriflavine. The corresponding results are provided on figure 3b.

### Purification with magnetic beads

The following samples were prepared with microbeads coated with Peps6, an ApoH protein derived peptide referenced in table 3 as ApoH. The corresponding results are provide in table 3 below:

**Table 3**

| | ApoH | ApoH |
|---|---|---|
| LoD Listeria (cells/ml) | 10² | 10²-10³ |
| LoD Salmonella (cells/ml) | 10¹-10² | 10² |
| Number of reagent | 2 | 4 |
| Easy to use | easy | easy |
| Time to result | Very fast | fast |
| Cost per assay | High | Medium |

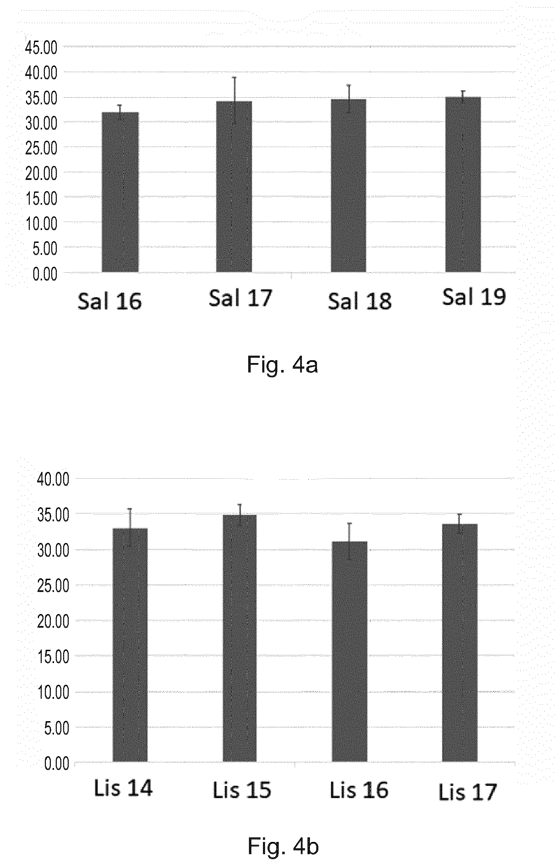

Salmonella was grown for 5 hours on swab and subsequently purified by either ApoH (Sal 16) or Rapiprep (Sal 17) microbeads and finally detected by pPCR, or on Meat and subsequently purified by either ApoH (Sal 18) or Rapiprep (Sal 19) microbeads and finally detected by qPCR. The corresponding results are provided on figure 4a.

Listeria was grown for 8 hours at 37°C on swab and subsequently purified by either ApoH (Lis 14) or Rapiprep (Lis 15) microbeads and finally detected by qPCR, or on Meat and subsequently purified by either ApoH (Lis 16) or Rapiprep (Lis 17) microbeads and finally detected by qPCR. The corresponding results are provided on figure 4b.

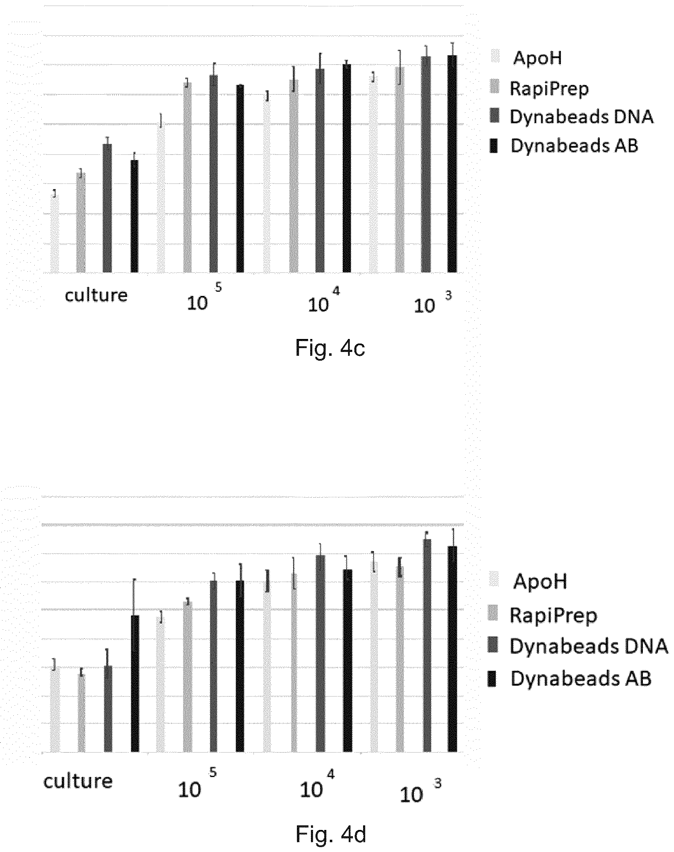

Figures 4c shows results of qPCR detection of Listeria monocytogenes from either an overnight culture, or various concentrations of 10⁵, 10⁴ and 10³ of defined bacteria numbers , using the following magnetic beads ApoH, RapiPrep, Dynabeads DNA, Dynabeads AB. The plots represents the average of 2 or 3 experiments, corresponding to 6 or 9 replicates.

Figure 4d shows the results of qPCR detection of Listeria monocytogenes from either an overnight culture, or various concentrations of 10⁵, 10⁴ and 10³ of defined bacteria numbers, using the following magnetic beads ApoH, RapiPrep, Dynabeads DNA, Dynabeads AB. The plots represents the average of 2 or 3 experiments, corresponding to 6 or 9 replicates.

### Buffering

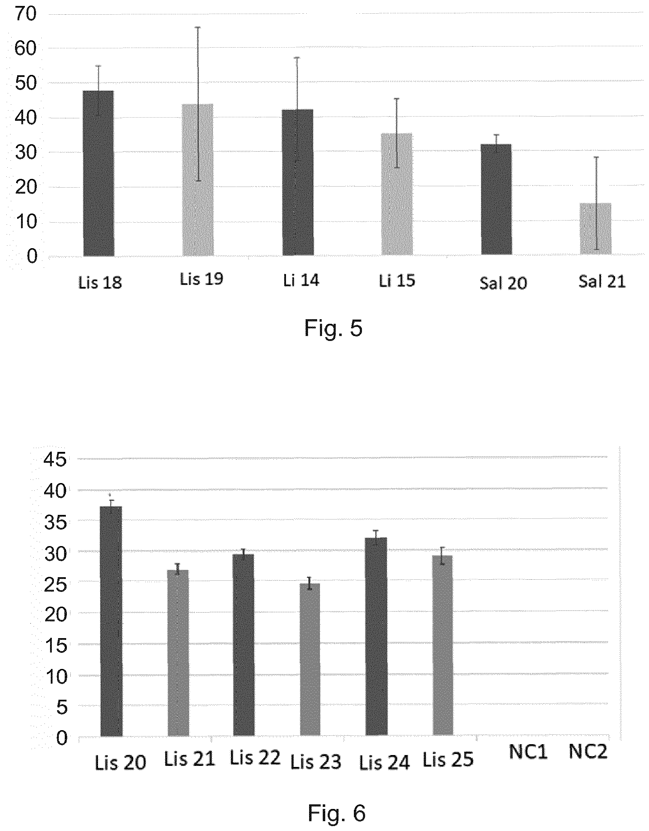

Figure 5 represents the capturing of Listeria monocytogenes in the presence TTGB (Lis 18) or AGT-1 (Lis 19) as buffering agent, Listeria ivanovii in the presence of TTGB (Li 14) or AGT-1 (Li 15) as buffering agent and Salmonella in the presence of TTGB (Sal 20) or AGT-1 (Sal 21) as buffering agent.

### Digesting experiments

Figure 6 shows detection results of Listeria monocytogenes after growing in meat + ½ Fraser without APA (Lis 20) or with APA (Lis 21), in Swab (BHI) without APA (Lis 22) or with APA (Lis 23), in swab (1/2 Fraser) without APA (Lis 24) or with APA (Lis 25), and a negative control without APA (NC1) and with APA (NC2),

Wherein APA denotes Bacillus subtilis Alkaline protease - Promega Catalogue Number A1721

The plots show an average of 3 experiments, corresponding to 9 replicates.

### 2 step detection

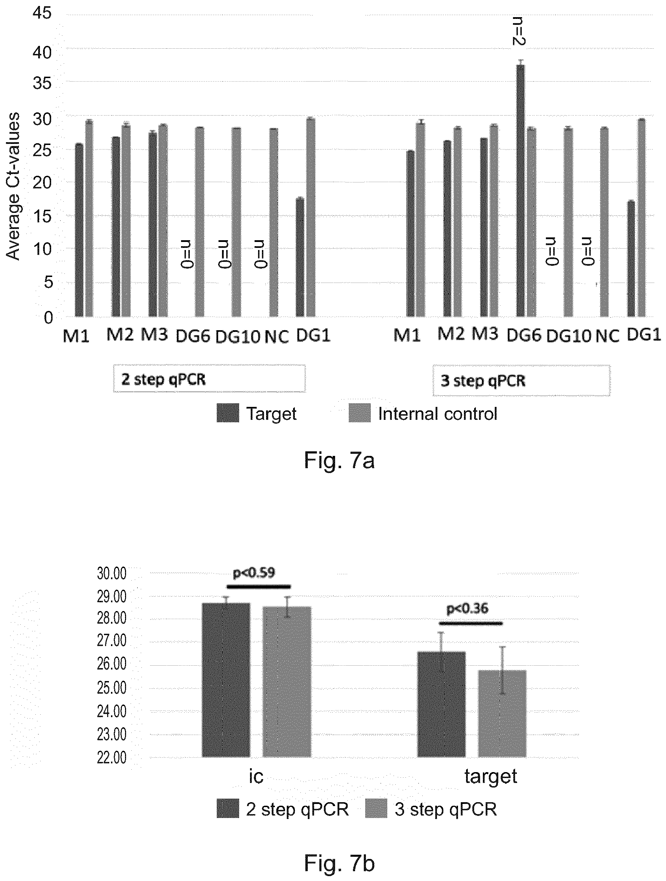

Figure 7a shows comparative results between 2 step detection and 3 step detection using a qPCR process for 3 samples (M1, M2, M3), a DG6 control sample, a DG10 control sample a negative control NC and a positive control DG1 sample,
Wherein
DG6 denotes Listeria monocytogenes
DG10 denotes Listeria ivanovii

Figure 7b shows comparative time of a so called fast (2step) and a so called slow (3 step) qPCR detection. The fast process allows for 30 minutes less time than a slow detection.

### Effect of the dye detection

Figure 8 shows results of detection of samples with and without orange food dye for a DG6 control, for 3 samples (N1, N2, N3) and for a negative control (NC). No significant change in the detection is observed.
food dyes are mixtures of :
- E104 (Quinoline Yellow WS), E110 (orange yellow S), E124 (Ponceau 4R), (E122 (Azorubine), E129 (Allura Red AC), E133 (Brilliant blue FCF) or
- different ratios of E104 (Quinoline Yellow WS), E110 (orange yellow S), E124 (Ponceau 4R)

The relative amount variation of the different components did not shown significant variation of the results, indicating that the above-food dyes have no negative effect of the qPCR detection.

### Effect of dilution in sample detection

Figure 9a shows detection results of Listeria monocytogenes DNA for samples having amount of DNA of 1000 pg, 100 pg, 10 pg, 1 pg, 0.1 pg and for a negative control (NC) with an internal control.

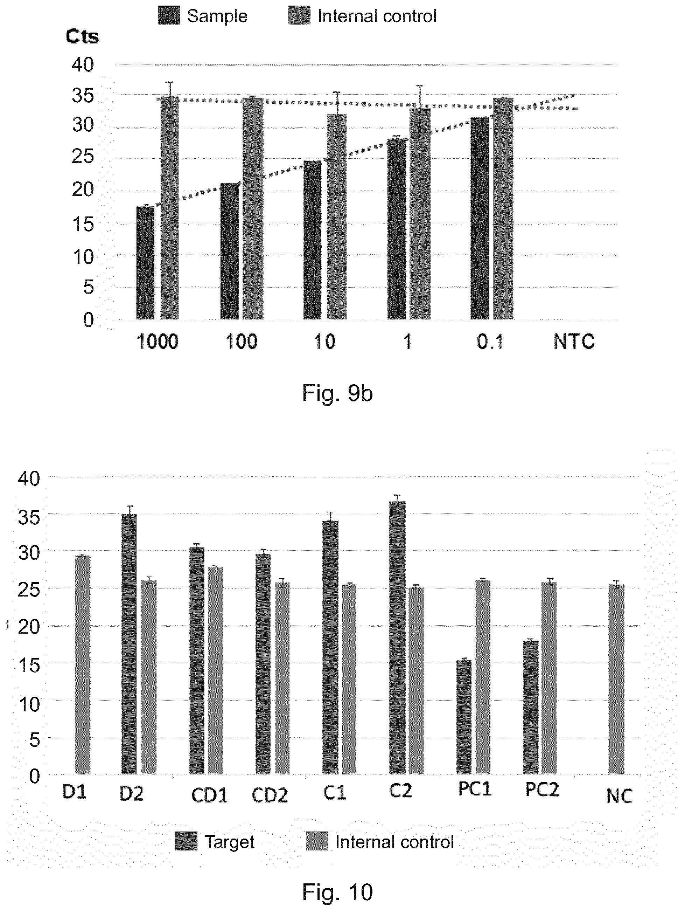

Figure 9b shows detection results of Salmonella's DNA for samples having amounts of DNA of 1000 pg, 100 pg, 10 pg, 1 pg, 0.1 pg and for a negative control (NC) with an internal control.

### Effect of an internal control

Figure 10 shows the effect of dilution on the ct values of meat lysate resulting from 22 hours in ½ Fraser enrichment broths.

Comparative results are provided for Duck without dilution (D1) and with 1/10 dilution (D2), for chicken drumstick without dilution (CD1) and with 1/10 dilution (CD2), for chicken without dilution (C1) and with 1/10 dilution (C2) for a positive control without dilution (PC1) and with 1/10 dilution (PC2), and for a negative control (NC). In case detection is not successful due to an elevated internal control, dilution can still be applied on the sample for an improved detection.

### Effect of BSA on the detection

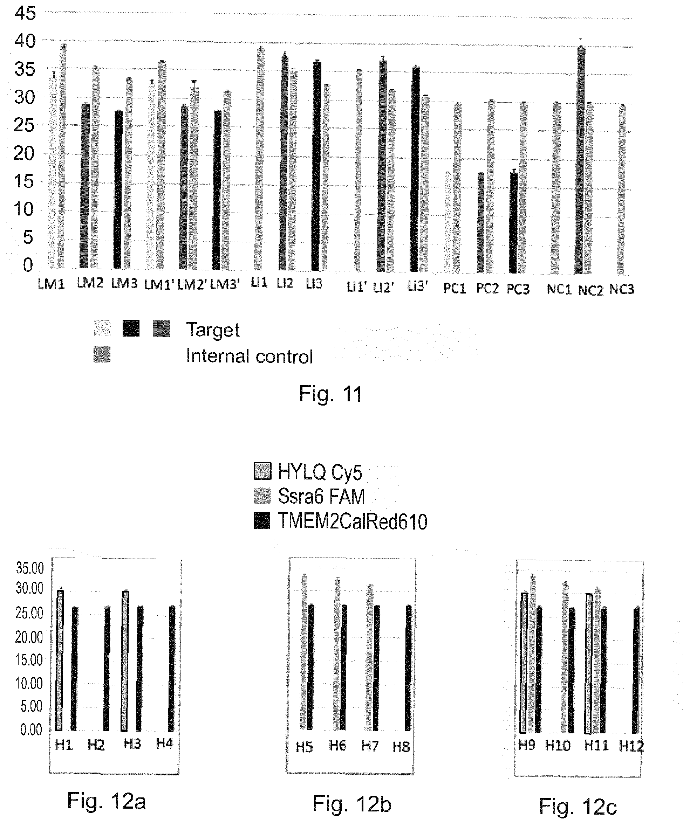

Figure 11 shows the effect of BSA concentration on the ct values of a sample after 14h in a ½ Fraser enrichment broth.

Three samples of Listeria monocytogenes are tested with 0,1 µg/µl of BSA (LM1), 0,5 µg/µl of BSA (LM2), 0,75 µg/µl of BSA (LM3). The experiment is iterated with another three samples under the same conditions (LM1', LM2', LM3')

Three samples of Listeria ivanorii are tested with 0,1 µg/µl of BSA (LI1), 0,5 µg/µl of BSA (LI2), 0,75 µg/µl of BSA (LI3).The experiment is iterated with three other samples under the same conditions (LI1', LI2', LI3').

Three samples of positive control are also tested with 0,1 µg/µl of BSA (PC1), 0,5 µg/µl of BSA (PC2), 0,75 µg/µl of BSA (PC3).

Three samples of negatives control are tested with 0,1 µg/µl of BSA (NC1), 0,5 µg/µl of BSA (NC2), 0,75 µg/µl of BSA (NC3).

### Multiplex detection

In figure 12a: HYLQ Cy5 was used to detect Listeria monocytogene alone DG6, 1pg (H1), Listeria ivanovii DG10, 1pg (H2), a mixture of Listeria monocytogene DG6 and Listeria ivanovii 1pg (H3), and in a negative control H4. Listeria Monocytogenes was detected only when Lysteria monocytogenes DNA was present.

In figure 12b: Ssra6 FAM was used to detect Listeria monocytogene alone DG6, 1pg (H5), Listeria ivanovii DG10, 1pg (H6), a mixture of Listeria monocytogene DG6 and Listeria ivanovii 1pg (H7), and in a negative control (H8). Ssra6 allows to detect both Listeria ivanovii and Listeria ivanovii.

In figure 12c: both HYLQ Cy5 and Ssra6 FAM were used to detect Listeria monocytogene alone DG6, 1pg (H9), Listeria ivanovii DG10, 1pg (H10), a mixture of Listeria monocytogene DG6 and Listeria ivanovii 1pg (H11), and in a negative control (H12). The combined use of hylQ and ssra6 Listeria monocytogenes allowed detection on Listeria monocytogenes and Listeria species if L. monocytogenes DNA was present. The combination detected only Listeria spp, when Listeria ivanovii DNA was present but not Listeria monocytogenes. Finally, the combination allowed detection of Listeria monocytogenes and Listeria spp, when Listeria monocytogenes and Listeria ivanovii DNA were present.

The internal control was TMEM2 CalRed 610.

### reference symbols in the figures

- M: Culture medium
- L: Liquid solution
- P1, P2: Pathogens
- Cn: Contaminating cells
- B: magnetic beads
- G: Genetic material
- S1: Enrichment step
- S2: Sample preparation step
- S21: Suspending step
- S22: Isolation step
- S23: Releasing step
- S24: Collection step
- S25: sedimentation step
- S3: Detection step
- S4: Computing step

## Claims

1. A method for detecting and/or quantifying at least one targeted known pathogen (P1, P2) in a sample, the method comprising :
- an enrichment step (S1) in a medium (M) adapted to specifically, semi-specifically or selectively increase the amount of said targeted known pathogen or pathogens (P1, P2) in a cell culture;
- a sample preparation step (S2) adapted to semi-specifically purify and concentrate said targeted known pathogen or pathogens (P1, P2), said sample preparation comprises the step (S21) of suspending the cells of said cell culture, enriched with targeted known pathogen or pathogens, in a liquid solution (L), a step (S22) of isolating said targeted known pathogen or pathogens from said liquid solution (L) and a step (S24) of collecting the genetic material (G) of said targeted known pathogen or pathogens (P1, P2); and
- a detection step (S3) including a real-time PCR (qPCR) detection, wherein said genetic material (G) is amplified using a primer set so as to provide data related to the presence and/or amount of at least one of the targeted known pathogens;
wherein said at least one targeted known pathogen is selected from a Listeria and/or a Salmonella or a combination thereof.

2. Method according to claim 1, wherein said medium (M) is selected among a mixture of ½ Fraser and tryptic soy Broth (TSB), a Brain Heart infusion Broth (BHI), and a buffered pepton water (BPW).

3. Method according to one of claims 1 and 2, wherein the medium (M) further comprises antibiotics non significantly active on said targeted known pathogen or pathogens (P1, P2) so as to selectively promote the growing of said targeted known pathogen or pathogens.

4. Method according to one of claims 1 to 3, wherein the step (S22) of isolating said targeted known pathogen or pathogens (P1, P2) from said liquid solution (L) includes contacting the content of said liquid solution (L) with a solid support adapted to semi-specifically bind said targeted known pathogen or pathogens (P1, P2) and collecting said solid support combined with said targeted known pathogen or pathogens (P1, P2).

5. Method according to claim 4, wherein said solid support comprises magnetic beads being coated with a protein, a protein fragment, a peptide, or a combination thereof, adapted to semi-specifically bind said targeted known pathogen or pathogens (P1, P2).

6. Method according to claim 5, wherein said protein, a protein fragment, a peptide, or a combination thereof allows a multiplexing of several of the targeted known pathogens.

7. Method according to one of claims 5 and 6, said protein, protein fragment, peptide, or combination thereof being a molecule derived from ApoH, such as Peps6.

8. Method according to one of claims 1 to 7, said step (S24) of collecting the genetic material (G) of said targeted known pathogen or pathogens (P1, P2) includes their lysis by means of a chemical, mechanical, thermal treatment or a combination thereof.

9. Method according to one of claims 1 to 8, wherein at least one of the enrichment step (S1), sample preparation step (S2) and detection step (S3) includes buffering conditions resulting from the presence of one or more buffer selected from AGT-1 and TTGB.

10. Method according to one of claims 1 to 9, wherein said sample is a food sample and wherein at least one of the enrichment step (S1) and sample preparation step (S2) comprises a step of digesting the food matrix by an appropriate digestive compound or composition, such as Trypsin or a protease like an alkaline protease (APA) or a combination thereof.

11. Method according to one of claims 1 to 10, said detection step (S3) comprises a short 2 step temperature program comprising a first temperature level wherein the temperature is comprised between 90°C and 100°C, followed by a second temperature level wherein the temperature is comprised between 55°C and 75°C, said 2 step temperature program having a global time comprised between 15 and 30 minutes.

12. Method according to one of claims 1 to 11, said detection step (S3) comprising one or more of:
- incorporating one or several dyes, each dye being selective or specific from one targeted known pathogen (P1, P2), so as to discriminate the different pathogens present in the sample and/or increase the visibility,
- detecting several samples having different dilution levels of purified DNA so as to detect low to high number of said targeted pathogen or pathogens,
- detecting an internal control probe used as a positive control,
So as to provide analytical data.

13. Method according to one of claims 1 to 12, further comprising a step (S4) of computing the analytical data resulting from said detection step (S3), wherein the collection of said analytical data is based on an automatic optical detection and wherein the computing of said analytical data is based on predetermined optical threshold and predetermined algorithms, so that a final detection result is provided.

14. A kit adapted to detect and/or quantify at least one targeted known pathogen (P1, P2) selected from a Listeria or a Salmonella, said kit comprising
- at least one enrichment media (M) adapted to specifically, semi-specifically or selectively enrich said targeted known pathogen or pathogens, and
- at least one discriminant agent adapted to semi-specifically isolate and/or purify said targeted known pathogen or pathogens (P1, P2),
said kit being adapted to collect and prepare a sample at a point of care so that it is ready for the further detection step including a real-time PCR (qPCR) operation.
